(19) Europäisches Patentamt
European Patent Office
Office européen des brevets

(11) Publication number: **0 095 091**
**B1**

(12) **EUROPEAN PATENT SPECIFICATION**

(45) Date of publication of patent specification: **29.04.87**

(51) Int. Cl.$^4$: **C 07 D 457/04**

(21) Application number: **83104635.4**

(22) Date of filing: **11.05.83**

(54) **Process for the preparation of dihydrolysergic acid esters.**

(30) Priority: **26.05.82 DE 3220200**

(43) Date of publication of application:
**30.11.83 Bulletin 83/48**

(45) Publication of the grant of the patent:
**29.04.87 Bulletin 87/18**

(84) Designated Contracting States:
**AT BE CH DE FR GB IT LI LU NL SE**

(56) References cited:
**CH-A- 452 538**
**FR-A-1 115 726**
**GB-A- 759 105**

**S. PATAI: "The chemistry of acid derivatives", part 1, *supplement* B, 1979, pages 418-419, John Wiley & Sons, New York, US**

**The file contains technical information submitted after the application was filed and not included in this specification**

(73) Proprietor: **SCHERING AKTIENGESELLSCHAFT**
**Berlin und Bergkamen**
**Müllerstrasse 170/178 Postfach 65 03 11**
**D-1000 Berlin 65 (DE)**

(72) Inventor: **Sauer, Gerhard, Dr.**
**Königsbacher Zeile 41A**
**D-1000 Berlin 28 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**EP 0 095 091 B1**

## Description

### Background of the Invention

The present invention relates to a process for preparing dihydro lysergic acid esters.

The dihydrolysergic acid esters and dihydroisolysergic acid esters producible according to this invention are intermediates for the production of pharmacologically active compounds and also of each other. Thus, the known delergotrile can be prepared; for example, from the dihydroisolysergic acid esters according to the method described by Stuetz [P. L. Stuetz et al., J. Med. Chem. *21*: 754 (1978)]. The known metergoline (benzyl-(N-1,6-dimethyl-8β-ergolinylmethyl carbaminate) can be prepared, e.g. from the dihydro lysergic acid esters (Bernardi et al., Gazz. *94* (1964) 936; NL—A—67 102 10).

In general, an acid amide is prepared from the ester. However, it is also possible to conduct the inverse reaction, i.e., the alcoholysis of an acid amide or a nitrile into the corresponding ester as described by S. Patai: "The chemistry of acid derivatives", part I, suppl. B (1979), pages 418—419, paragraph 6. For this purpose, the acid amide is dissolved in an alcohol and a strong acid is added thereto. In most cases, a high acid concentration is required, for example, by introducing hydrogen chloride for several hours. This reaction is ordinarily accomplished under boiling heat of the alcohol employed, in some cases even in a sealed tube (Weyand-Hilgetag, "Organisch-chemische Experimentierkunst" [Experimenting Art in Organic Chemistry] Leipzig 1970, p. 352.

The reaction of the corresponding nitrile with an alcohol is described to yield the alkyl ester in FR—A—1 115 726 and GB—A—759 105.

On the other hand, lysergic acid and its derivatives have low stability under heat and under stongly acidic conditions. This poses a major problem in acid catalyzed reactions preparing or using such esters.

Alkaline saponification of dihydrolysergic acid amides likewise takes place under rather drastic conditions with 4—7N potassium hydroxide solution under heating, and does not yield pure dihydrolysergic acid which then would first have to be esterified in a second step DE—B—2,610,859).

### Summary of the Invention

It is an object of the present invention to provide a process that produces the desired dihydrolysergic acid esters in high yield and purity.

It has now been found surprisingly that it is possible to convert 8α-dihydrolysergic acid amides, in the presence of an inorganic or organic acid, into the corresponding ester in a smooth reaction. There is neither a decomposition of dihydrolysergic acid nor an isomerization in the 8-position. The 8α-dihydro lysergic acid amides, readily conventionally producible in the pure form, result in the 8α-dihydrolysergic acid esters, respectively, in an almost quantitative yield (e.g., typically at least about 75 molar % of theory).

Upon further study of the specification and appended claims, further objects and advantages of this invention will become apparent to those skilled in the art.

These objects have been attained by providing a process for the preparation of dihydro lysergic acid esters of the formula

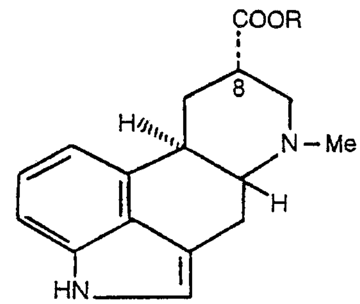

wherein

R is alkyl of up to 3 carbon atoms, and

the substituent in the 8-position is in the α-position, comprising reacting the corresponding acid amides of the formula

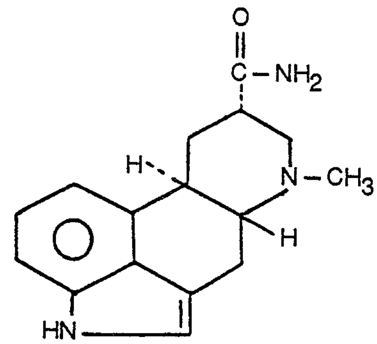

or a reaction compatible salt thereof with an acid

with an alcohol of the formula ROH wherein R is an alkyl residue of up to 3 carbon atoms, at temperatures of 20° to 55°C for a period of 50—8 hours in the presence of an acid or an acidic ion exchange resin at a pH value of 0—1.

### Detailed Discussion

In order to conduct the process of this invention, the dihydrolysergic acid amide utilized as the starting material is dissolved, e.g., in methanol, ethanol, n-propanol, or isopropanol. Generally, amounts of the starting material amide relative to the alcohol are 1—100 parts by weight, i.e., usually an excess of alcohol is utilized. Suitable acids include sulfuric acid, hydrochloric acid, perchloric acid, p-toluenesulfonic acid, trifluoroacetic acid, trifluoromethanesulfonic acid, etc., or an acidic ion exchange resin, e.g., sulfonic acids like $RSO_3H$, wherein R is the resin residue.

The acid is added to the alcohol solution in a quantity so that the pH value of the reaction solution is 0—1. Thereafter the reaction solution is maintained in the temperature range of 20—55°C. Depending on the acid concentration, the reaction period is at least 8 hours but at most

50 hours and, in the normal case, is completed after 20 hours.

The starting material can also be a reaction compatible salt of the amide with an acid, e.g., hydrogen phosphate, tartrate or hydrogen maleate. Preferably, the reaction is conducted under anhydrous conditions.

The reaction mixture is subsequently worked up by using known methods such as neutralization, washing, extraction, precipitation, crystallization, chromatography, etc.

Without further elaboration, it is believed that one skilled in the art can, using the preceding description, utilize the present invention to its fullest extent. The following preferred specific embodiments are, therefore, to be construed as illustrative. In the following examples, all temperatures are set forth uncorrected in degrees Celsius; unless otherwise indicated, all parts and percentages are by weight.

Example 1

A solution is prepared from 3.32 g of 6-methylergoline-8α-carboxylic acid amide (12 millimoles) in 350 ml of anhydrous methanol; 300 g of ion exchanger "Amberlite" IR 120 (acidic form, washed out with anhydrous methanol) is added thereto, and the mixture is allowed to stand for 2 days at 50°C is a sealed flask. The content of the flask is then transferred into a glass column and eluted with 1.5 l of a mixture of methanol, triethylamine, and water (55 : 25 : 20). After the solvent has been removed by distillation, the residue is crystallized from methanol. Yield: 3.0 g (85% of theory) of the methyl ester of 6-methylergoline-8α-carboxylic acid.

$[\alpha]_D = -81°$ (0.5% in pyridine).

Example 2

A solution is prepared from 18.76 g of 6-methylergoline-8α-carboxylic acid amide and 500 g of p-toluenesulfonic acid in 2 l of anhydrous methanol, and the solution is stirred overnight at 50°C. Half of the solvent is then removed by distillation, the residue is poured into a mixture of 200 ml of concentrated ammonia and ice and extracted with methylene chloride. The organic phase is dried with sodium sulfate, evaporated, and crystallized from methanol. Yield: 17.2 g (87% of theory) of the methyl ester of 6-methylergoline-8α-carboxylic acid.

$[\alpha]_D = -81°$ (0.5% in pyridine).

The preceding examples can be repeated with similar success by substituting the generically or specifically described reactants and/or operating conditions of this invention for those used in the preceding examples.

From the foregoing description, one skilled in the art can easily ascertain the essential characteristics of this invention.

**Claims**

1. A process for preparing a dihydro lysergic acid ester of the formula

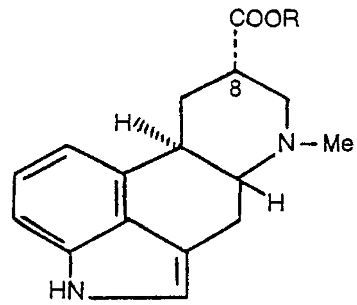

wherein R is alkyl of up to 3 carbon atoms, and the substituent in the 8-position is in the α-position, comprising reacting a corresponding acid amide of the formula

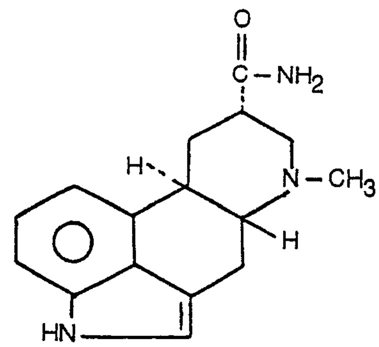

or a reaction compatible salt thereof with an acid, with an alcohol of the formula ROH wherein R is alkyl of up to 3 carbon atoms, at a temperature of 20° to 55°C for a period of 8 to 50 hours in the presence of an acid or an acidic ion exchange resin at a pH value of 0—1.

2. A process of claim 1 wherein the pH is provided by adding an acid.

3. A process of claim 1 wherein the pH is effectively provided by using an acidic ion exchange resin.

4. A process of claim 1 wherein the acid is HCl or p-toluenesulfonic acid.

5. A process of claim 1 wherein the reaction time is 8 to 20 hours.

**Patentansprüche**

1. Ein Verfahren zur Herstellung eines Dihydrolysergsäureesters der Formel

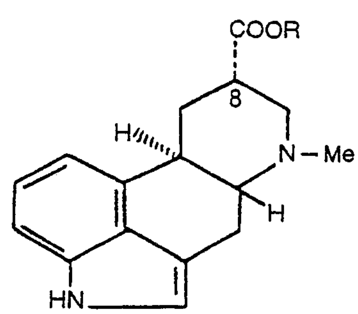

worin R Alkyl mit bis zu 3 Kohlenstoffatomen ist, und der Substituent in der 8-Stellung in der α-

Stellung vorliegt, umfassend das Umsetzen eines entsprechenden Säureamids der Formel

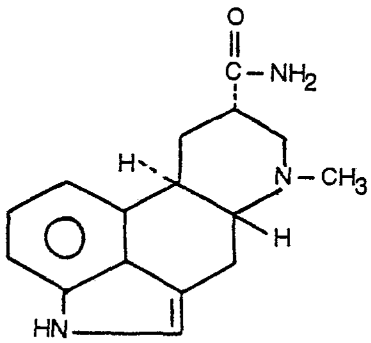

oder eines reaktionsverträglichen Salzes davon, mit einer Säure, mit einem Alkohol der Formel ROH, worin R Alkyl mit bis zu 3 Kohlenstoffatomen ist, bei einer Temperatur von 20° bis 55°C während eines Zeitraumes von 8 bis 50 Stunden in Gegenwart einer Säure oder eines sauren Ionenaustauscherharzes bei einem pH-Wert von 0—1.

2. Ein Verfahren des Anspruchs 1, wobei der pH-Wert durch Zugabe einer Säure bestimmt wird.

3. Ein Verfahren des Anspruchs 1, wobei der pH-Wert durch Verwendung eines sauren Ionenaustauscherharzes wirksam bestimmt wird.

4. Ein Verfahren des Anspruchs 1, wobei die Säure HCl oder p-Toluolsulfonsäure ist.

5. Ein Verfahren des Anspruchs 1, wobei die Reaktionszeit 8 bis 20 Stunden beträgt.

**Revendications**

1. Procédé de préparation d'un ester de l'acide dihydro-lysergique répondant à la formule:

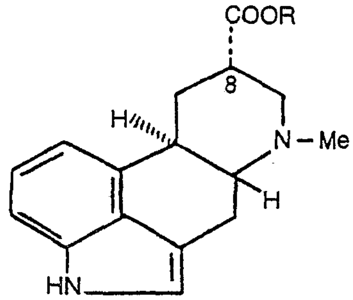

dans laquelle R représente un radical alkyle contenant au plus 3 atomes de carbone et dans laquelle le substituant en position 8 a la configuration α, procédé selon lequel on fait réagir un carboxamide correspondant de formule:

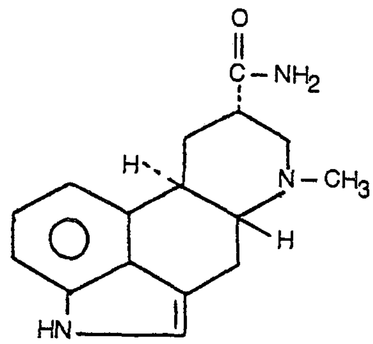

(ou un sel compatible avec la réaction dérivant de ce composé et d'un acide) avec un alcool de formule ROH dans lequel R représente un radical alkyle contenant au plus 3 atomes de carbone, à une température de 20 à 55°C, pendant une durée de 8 à 50 heures, en présence d'un acide ou d'une résine échangeuse d'ions acide, à un pH de 0 à 1.

2. Procédé selon la revendication 1 caractérisé en ce que le pH est réglé à la valeur voulue par l'addition d'un acide.

3. Procédé selon la revendication 1 caractérisé en ce que le pH voulu est réglé efficacement au moyen d'une résine échangeuse d'ions acide.

4. Procédé selon la revendication 1 caractérisé en ce que l'acide est HCl ou l'acide p-toluènesulfonique.

5. Procédé selon la revendication 1 caractérisé en ce que la durée de la réaction est de 8 à 20 heures.